Europäisches Patentamt

European Patent Office

Office européen des brevets

(11) Numéro de publication: **0 251 952 B1**

# FASCICULE DE BREVET EUROPEEN

(45) Date de publication de fascicule du brevet: **30.09.92**  (51) Int. Cl.⁵: **C07C 275/62**, C08G 18/78

(21) Numéro de dépôt: **87420184.1**

(22) Date de dépôt: **01.07.87**

(54) **Procédé de préparation de polyisocyanate à groupement biuret.**

(30) Priorité: **04.07.86 FR 8609947**

(43) Date de publication de la demande:
**07.01.88 Bulletin 88/01**

(45) Mention de la délivrance du brevet:
**30.09.92 Bulletin 92/40**

(84) Etats contractants désignés:
**AT BE CH DE ES FR GB GR IT LI LU NL SE**

(56) Documents cités:
**EP-A- 0 157 088**

(73) Titulaire: **RHONE-POULENC CHIMIE**
**25, quai Paul Doumer**
**F-92408 Courbevoie Cédex(FR)**

(72) Inventeur: **Robin, Jean**
**21, rue Duguesclin**
**F-69006 Lyon(FR)**

(74) Mandataire: **Vignally, Noel et al**
**Rhône-Poulenc Interservices Service Brevets Chimie Centre de Recherches des Carrières B.P. 62**
**F-69192 Saint-Fons Cédex(FR)**

Rank Xerox (UK) Business Services

## Description

La présente invention concerne un procédé de préparation de polyisocyanate à groupement biuret, plus particulièrement à partir d'un diisocyanate aliphatique, cycloaliphatique ou arylaliphatique et d'eau.

Les polyisocyanates à groupement biuret peuvent être utilisés pour préparer des mousses, des adhésifs et des peintures.

Parmi ces applications, l'utilisation de ces polyisocyanates comme constituants des peintures devient de plus en plus importante, notamment pour l'industrie automobile.

Les pellicules de peintures préparées à partir de polyisocyanates aromatiques jaunissent et se fendillent.

Par contre, les pellicules et peintures préparées à partir de polyisocyanates aliphatiques, cycloaliphatiques ou arylaliphatiques conservent leurs propriétés pendant de très longues durées et sont donc particulièrement adaptées pour les véhicules automobiles.

Cependant, la préparation des polyisocyanates aliphatiques, cycloaliphatiques et arylaliphatiques, au moyen de diisocyanate et d'eu, présente certains inconvénients.

Ainsi, selon les brevets américains No. 3.124.605 et 3.902.127, la réaction du diisocyanate monomère et de l'eau produit de la polyurée qui précipite. La réaction entre le diisocyanate monomère et l'eau qui se dissout en faibles quantités dans ledit diisocyanate, produit le polyisocyanate à groupement biuret. Mais la réaction de l'eau avec les faibles quantités de diisocyanate monomère qu'elle peut dissoudre, conduit à de la polyurée.

Dans le brevet français publié sous le numéro 2.382.468, il a été proposé, comme solution à ce problème, de préparer ces polyisocyanates par reaction du diisocyanate monomère sur l'eau, à une température d'au moins 70°C et dans un mélange d'un dérivé de l'éthylèneglycol, tel qu'un éther méthylique de l'acétate d'éthylèneglycol, et d'un phosphate de méthyle ou d'éthyle.

Ce procédé permet de supprimer pratiquement la précipitation de polyurée dans le polyisocyanate.

Mais il présente l'inconvénient de mettre en oeuvre des quantités importantes de solvant, ce qui diminue la productivité de l'appareillage et complique les opérations ultérieures de distillation.

Il est également connu selon EP-A 0 157 088 un procédé de préparation de polyisocyanates à groupement biuret qui consiste à faire réagir un diisocyanate aliphatique à température élevée et en présence d'un acide acétique $\alpha,\alpha,\alpha$-trisubstitué ou son anhydrique et, plus particulièrement, l'acide pivalique. Ledit procédé reguiert de manière impérative la présence d'un acide acétique $\alpha,\alpha,\alpha$-trisubstitué.

La demanderesse a trouvé un nouveau procédé de préparation de polyisocyanates à groupement biuret qui ne présente pratiquement pas les inconvénients indiqués précédemment.

Ce procédé consiste à faire réagir au moins un diisocyanate monomère aliphatique, cycloaliphatique ou arylaliphatique et de l'eau en l'absence de tout acide acétique $\alpha,\alpha,\alpha$-trisubstitué ou de ses anhydrides, et est caractérisé en ce que l'on opère à une température au moins égale à 70°C et sous une pression absolue au moins égale à 1,2 bar dont une pression partielle de gaz carbonique au moins égale à 0,2 bar.

On peut, soit avoir une pression égale à la pression atmosphérique d'un gaz inerte vis-à-vis des réactifs tel que l'azote, l'argon ou l'air secs, complétée par une pression partielle de gaz carbonique égale ou supérieure à 0,2 bar, soit effectuer une purge par du gaz carbonique et avoir une pression initiale égale ou supérieure à 1 bar absolu.

On peut laisser croître la pression autogène de la réaction, due à la formation de gaz carbonique, pendant toute la réaction ou la maintenir à la valeur souhaitée en laissant échapper une partie du $CO_2$ formé.

Bien que l'utilisation de pressions très élevées ne présente guère d'intérêt pratique, on peut mettre en oeuvre le procédé selon l'invention sous des pressions partielles de gaz carbonique de 0,2 à 100 bars.

De préférence cependant, la pression partielle de gaz carbonique sera généralement comprise entre 0,5 et 50 bars, la pression totale étant supérieure ou égale à 1,5 bar absolu.

Les diisocyanates monomères qui peuvent être utilisés dans le présent procédé sont les diisocyanates aliphatiques, les diisocyanates cycloaliphatiques et les diisocyanates arylaliphatiques dont les groupements isocyanate ne sont pas directement liés à un cycle aromatique.

A titre d'exemples non limitatifs de ces diisocyanates monomère, on peut citer :
- le diisocyanato-1,3 propane,
- le diisocyanato-1,4 butane,
- le diisocyanato-1,5 pentane,
- le diisocyanato-1,6 hexane,
- le diisocyanato-1,4 éthyl-2 butane,
- le diisocyanato-1,5 méthyl-2 pentane,

- le diisocyanato-1,6 triméthyl-2,2,4 hexane,
- le diisocyanato-1,6 triméthyl-2,4,4 hexane,
- le diisocyanato-1,2 cyclohexane,
- le diisocyanato-1,4 cyclohexane,
- le bis(isocyanatométhyl)-1,2 cyclobutane,
- le bis(isocyanato-4 cyclohexyl)méthane,
- le triméthyl-3,3,5 isocyanatométhyl-5 isocyanato-1 cyclohexane,
- le bis(isocyanatométhyl)-1,4 benzène,
- le bis(isocyanatométhyl)-1,2 benzène.

Les diisocyanates monomères peuvent être utilisés séparément ou sous forme de mélanges de plusieurs d'entre eux.

Ainsi par exemple le diisocyanato-1,6 hexane, qui est l'un des diisocyanates monomères préférés, peut être utilisé seul ou en mélanges, notamment avec le diisocyanato-1,5 méthyl-2 pentane et/ou le diisocyanato-1,4 éthyl-2 butane ; des mélanges de ces deux derniers diisocyanates peuvent également être utilisés.

Le rapport molaire diisocyanate monomère/eau peut varier dans de très larges limites. Cependant la masse moléculaire du polyisocyanate obtenu est d'autant plus élevée que le rapport molaire diisocyanate monomère/eau est plus bas. Généralement il n'est pas favorable d'avoir une masse moléculaire et une viscosité trop élevées.

D'autre part la masse moléculaire et la viscosité du polyisocyanate obtenu sont d'autant plus faibles que le rapport diisocyanate monomère/eau est plus élevé. Mais un rapport trop élevé n'est pas intéressant économiquement, car cela implique la séparation et la récupération d'une quantité importante de diisocyanate monomère.

C'est pourquoi on adopte généralement un rapport molaire diisocyanate monomère/eau compris entre 2 et 40. De préférence ce rapport est de 5 à 15.

La température, à laquelle est opérée la réaction du diisocyanate monomère et de l'eau, est en général comprise entre 70°C et 200°C. L'utilisation de température plus élevée ne gêne pas la réaction, mais elle conduit à des colorations indésirables.

De préférence on opère entre 110°C et 150°C.

Le procédé selon l'invention peut être mis en oeuvre en présence ou en l'absence de solvant.

Si l'on opère sans solvant, il est nécessaire d'avoir une agitation suffisante pour avoir un bon contact entre le diisocyanate monomère et l'eau.

Les résultats obtenus sont très satisfaisants ; le biuret obtenu est limpide.

On peut également, et c'est une autre variante du présent procédé, utiliser de faibles quantités de solvant pour diluer l'eau et favoriser son contact et donc sa réaction avec le diisocyanate monomère.

Par faibles quantités de solvant, on entend des quantités représentant de 1 à 10 fois le poids de l'eau engagée. De préférence ces quantités représentent de 1 à 5 fois le poids de l'eau engagée. Ces chiffres n'ont qu'une valeur indicative, car on peut également sans problème mettre en oeuvre la réaction en milieu solvant, c'est-à-dire par exemple avec une quantité de solvant représentant en poids de 10 % à 80 % du mélange réactionnel.

Les résultats obtenus en milieu solvant sont excellents, mais bien entendu, on retrouve l'inconvénient lié à une moindre productivité de l'appareillage et à la nécessité d'opérations supplémentaires de séparation.

Le solvant, lorsqu'il est présent en faible quantité ou en quantité plus importante, peut être un alkoxy-alcane ou un carboxylate d'alkoxy-alcane dérivant par exemple de l'éthylèneglycol (ou de l'oxyde d'éthylène), du propylèneglycol (ou de l'oxyde de propylène) et éventuellement d'un acide carboxylique tel que l'acide acétique.

A titre d'exemples, on peut citer les dérivés de l'éthylèneglycol décrits dans le brevet français publié sous le numéro 2.382.468.

On peut utiliser plus particulièrement l'acétate de méthoxy-2 éthyle, le diméthoxy-1,2 éthane, l'acétate d'éthoxy-2 éthyle, le diacétate de l'éthylèneglycol, l'acétate de méthoxy-1 propyle-2, l'acétate de méthoxy-2 propyle-1, l'acétate d'éthoxy-1 propyle-2, l'acétate d'éthoxy-2 propyle-1.

Le solvant utilisé peut être également un ester méthylique et/ou éthylique de l'acide phosphorique, notamment le phosphate de triméthyle, le phosphate de triéthyle, le phosphate de diméthyle et d'éthyle ou le phosphate de diéthyle et de méthyle.

Ces solvants, lorsqu'ils sont employés, peuvent l'être séparément ou en mélanges tels que par exemple des mélanges d'un alkoxy-alcane ou d'un carboxylate d'alkoxy-alcane avec un ester phosphorique.

En pratique on peut réaliser le procédé selon l'invention en chargeant tout d'abord le diisocyanate monomère ou le mélange de diisocyanates ; on purge l'appareillage par un gaz inerte sec tel que l'azote,

EP 0 251 952 B1

l'argon ou par du gaz carbonique. On ajoute alors sous agitation l'eau diluée par 1 à 10 fois son poids de l'un des solvants indiqués précédemment.

On met l'appareillage sous une pression de 2 à 10 bars absolus à l'aide de gaz carbonique et on chauffe vers 120° à 140°C. La pression est généralement maintenue à une valeur de 2 à 10 bars absolus par élimination de l'excès de $CO_2$.

Après la fin de la réaction (quelques minutes à quelques heures), on dégaze le gaz carbonique et on élimine l'excès de diisocyanate monomère. On obtient un biuret limpide sans précipité. Sa viscosité est fonction du rapport molaire diisocyanate/eau adopté.

Le biuret est stable dans le temps ; on ne note pas de précipitation après plusieurs mois de stockage, ni d'augmentation sensible de taux de diisocyanate libre.

Les exemples qui suivent illustrent l'invention.

EXEMPLES 1 à 5

Dans un autoclave de 3,6 litres, muni d'un agitateur en forme d'hélice et chauffé par une double enveloppe à circulation, on charge du diisocyanato-1,6 hexane (HDI) et éventuellement des solvants.

L'eau est introduite dans l'HDI au moyen d'une pompe doseuse à débit réglable de 0 à 600 ml/h, connectée à un tube plongeant. Le gaz formé se dégage par un réfrigérant à reflux, surmonté d'un manomètre et d'une soupape tarée réglable, permettant de maintenir l'autoclave sous une pression constante choisie au préalable.

On chauffe l'HDI (et éventuellement les solvants) à 130°C et on ajoute l'eau à la vitesse constante de 1 ml/min. Après addition de la quantité d'eau requise, on maintient la température à 130°C pendant 3 heures.

Vers la fin de l'addition de l'eau, la pression monte et atteint la valeur choisie. La soupape réglable s'ouvre alors, laisse passer le gaz excédentaire et maintient dans l'autoclave la pression choisie pour l'essai.

En fin d'opération, on ramène l'autoclave à pression atmosphérique, on refroidit et on soutire le produit obtenu : mélange d'HDI, de biuret et éventuellement des solvants.

Ce produit est ensuite évaporé au moyen d'un évaporateur à film agité.

Le tableau ci-après rassemble les données et les résultats des différents exemples.

4

| | Exemple 1 | Exemple 2 | Exemple 3 | Exemple 4 | Exemple 5 |
|---|---|---|---|---|---|
| HDI | 1508 g | 2262 g | 2262 g | 2262 g | 2262 g |
| eau | 20 g | 30 g | 30 g | 30 g | 30 g |
| solvants (1) | 650 g | – | – | – | – |
| pression absolue | 6 bars | 6 bars | 4 bars | 2 bars | 1,3 bar |
| viscosité du biuret à 25° | 8,9 Pa.s | 4,2 Pa.s | 3,6 Pa.s | 3,3 Pa.s | 4,3 Pa.s |
| HDI % en poids en fin d'essai | (*) 0,05 | (*) 0,05 | (*) 0,05 | (*) 0,05 | (*) 0,05 |
| stabilité(2) HDI % en poids | 0,2 | 0,2 | 0,2 | 0,2 | 0,2 |

(1) = mélange 1/1 en poids d'acétate de méthoxyéthyle et de phosphate d'éthyle.

(2) = après stockage d'un mois à 60°C.

(*) = limite de précision du dosage.

On constate qu'en opérant sans solvant le biuret obtenu est moins visqueux (exemple 2) qu'en opérant avec des solvants (exemple 1).

Dans tous les exemples le produit obtenu enfin de réaction et le biuret final sont limpides.

CONTRE-EXEMPLE A

5

En effectuant la réaction de biurétisation dans les mêmes conditions que pour les exemples 2 à 5 (sans solvant), mais en opérant sous pression atmosphérique, le produit obtenu est très trouble avec un précipité de polyurée.

HDI % en poids en fin d'essai : 0,15 % ;

HDI % en poids après stockage d'un mois à 60°C : 0,8 %.

## Revendications

1. Procédé de préparation d'un polyisocyanate organique à groupement biuret consistant à faire réagir au moins un diisocyanate aliphatique, cycloaliphatique ou arylaliphatique et de l'eau, en l'absence de tout acide acétique $\alpha,\alpha,\alpha$-trisubstitué ou de ses anhydrides, caractérisé en ce que l'on opère à une température d'au moins 70°C et sous une pression absolue au moins égale à 1,2 bar dont une pression partielle de gaz carbonique au moins égale à 0,2 bar.

2. Procédé selon la revendication 1, caractérisé en ce que la pression partielle de gaz carbonique est comprise entre 0,5 et 50 bars et la pression absolue supérieure ou égale à 1,5 bar.

3. Procédé selon l'une des revendications 1 ou 2, caractérisé en ce que le rapport molaire diisocyanate monomère/eau est compris entre 2 et 40 et de préférence entre 5 et 15.

4. Procédé selon l'une des revendications 1 à 3, caractérisé en ce que l'on n'utilise pas de solvant.

5. Procédé selon l'une des revendications 1 à 3, caractérisé en ce que l'on opère en présence d'un ou plusieurs solvants tels que les alkoxy-alcanes, les carboxylates d'alkoxy-alcanes et les esters méthyliques et/ou éthyliques de l'acide phosphorique.

6. Procédé selon la revendication 5, caractérisé en ce que le poids de solvant utilisé représente de 1 à 10 fois le poids de l'eau engagée et de préférence de 1 à 5 fois ce poids.

7. Procédé selon la revendication 5, caractérisé en ce que le poids de solvant utilisé représente de 10 à 80 % du poids du mélange réactionnel.

8. Procédé selon l'une des revendications 5 à 7, caractérisé en ce que les solvants sont choisis parmi l'acétate de méthoxy-2 éthyle, le diméthoxy-1,2 éthane, l'acétate d'éthoxy-2 éthyle, le diacétate de l'éthylèneglycol, l'acétate de méthoxy-1 propyle-2, l'acétate de méthoxy-2 propyle-1, l'acétate d'éthoxy-1 propyle-2, l'acétate d'éthoxy-2 propyle-1 ou un ester méthylique et/ou éthylique de l'acide phosphorique, tel que le phosphate de triméthyle, le phosphate de triéthyle, le phosphate de diméthyle et d'éthyle ou le phosphate de diéthyle et de méthyle.

9. Procédé selon l'une des revendications 1 à 8, caractérisé en ce que la réaction est effectuée entre 70°C et 200°C et de préférence entre 110°C et 150°C.

## Claims

1. Process for the preparation of an organic polyisocyanate containing a biuret group, consisting in reacting at least one aliphatic, cycloaliphatic or arylaliphatic diisocyanate with water, in the absence of any $\alpha,\alpha,\alpha$-trisubstituted acetic acid or its anhydrides, characterised in that the operation is carried out at a temperature of at least 70°C and at an absolute pressure at least equal to 1.2 bar, including a partial pressure of carbon dioxide at least equal to 0.2 bar.

2. Process according to Claim 1, characterised in that the partial pressure of carbon dioxide is between 0.5 and 50 bars and the absolute pressure is greater than or equal to 1.5 bar.

3. Process according to either of Claims 1 and 2, characterised in that the molar ratio of monomeric diisocyanate to water is between 2 and 40 and preferably between 5 and 15.

4. Process according to one of Claims 1 to 3, characterised in that no solvent is used.

EP 0 251 952 B1

**5.** Process according to one of Claims 1 to 3, characterised in that it is carried out in the presence of one or more solvents such as alkoxyalkanes, alkoxyalkane carboxylates and methyl and/or ethyl esters of phosphoric acid.

**6.** Process according to Claim 5, characterised in that the weight of solvent used represents from 1 to 10 times the weight of the water employed and preferably from 1 to 5 times this weight.

**7.** Process according to Claim 5, characterized in that the weight of solvent used represents from 10 to 80% of the weight of the reaction mixture.

**8.** Process according to one of Claims 5 to 7, characterized in that the solvents are chosen from 2-methoxyethyl acetate, 1,2-dimethoxyethane, 2-ethoxyethyl acetate, ethylene glycol diacetate, 1-methoxy-2-propyl acetate, 2-methoxy-1-propyl acetate, 1-ethoxy-2-propyl acetate, 2-ethoxy-1-propyl acetate or a methyl and/or ethyl ester of phosphoric acid, such as trimethyl phosphate, triethyl phosphate, dimethyl ethyl phosphate or diethyl methyl phosphate.

**9.** Process according to one of Claims 1 to 8, characterised in that the reaction is carried out between 70°C and 200°C and preferably between 110°C and 150°C.

**Patentansprüche**

**1.** Verfahren zur Herstellung eines organischen Polyisocyanats mit Biuretstruktur, bei dem mindestens ein aliphatisches, cycloaliphatisches oder arylaliphatisches Diisocyanat mit Wasser in Abwesenheit einer $\alpha,\alpha,\alpha$-trisubstituierten Essigsäure oder deren Anhydriden umgesetzt wird, dadurch gekennzeichnet, daß man bei einer Temperatur von mindestens 70°C und unter einem Gesamtdruck von mindestens 1,2 bar arbeitet, wobei der Partialdruck des Kohlendioxyds mindestens gleich 0,2 bar beträgt.

**2.** Verfahren nach Anspruch 1, dadurch gekennzeichnet, daß der Partialdruck des Kohlendioxids zwischen 0,5 und 50 bar liegt und der Gesamtdruck höher oder gleich 1,5 bar ist.

**3.** Verfahren nach einem der Ansprüche 1 und 2, dadurch gekennzeichnet, daß das Molverhältnis von monomeren Diisocyanat zu Wasser zwischen 2 und 40, vorzugsweise 5 und 15 liegt.

**4.** Verfahren nach einem der Ansprüche 1 bis 3, dadurch gekennzeichnet, daß man kein Lösungsmittel verwendet.

**5.** Verfahren nach einem der Ansprüche 1 bis 3, dadurch gekennzeichnet, daß man in Gegenwart eines oder mehrerer Lösungsmittel wie Alkoxyalkanen, Alkoxyalkancarboxylaten und Phosphorsäuremethyl- und/oder -ethylester arbeitet.

**6.** Verfahren nach Anspruch 5, dadurch gekennzeichnet, daß das Gewicht des verwendeten Lösungsmittels das 1 bis 10, vorzugsweise 1 bis 5 fache des Gewichts des eingesetzten Wassers beträgt.

**7.** Verfahren nach Anspruch 5, dadurch gekennzeichnet, daß das Gewicht des verwendeten Lösungsmittels 10 bis 80 Gew.% des Reaktionsgemisches darstellt.

**8.** Verfahren nach einem der Ansprüche 5 bis 7, dadurch gekennzeichnet, daß die Lösungsmittel aus 2-Methoxyethylacetat, 1,2-Dimethoxyethan, 2-Ethoxyethylacetat, Ethylenglykoldiacetat, 1-Methoxypropyl-2-acetat, 2-Methoxypropyl-1-acetat, 1-Ethoxypropyl-2-acetat, 2-Ethoxypropyl-1-acetat oder Phosphorsäuremethyl- und/oder -ethylester wie Trimethylphosphat, Triethylphosphat, Dimethyl-ethylphosphat und Diethyl-methylphosphat ausgewählt werden.

**9.** Verfahren nach einem der Ansprüche 1 bis 8, dadurch gekennzeichnet, daß die Umsetzung zwischen 70°C und 200°C, vorzugsweise zwischen 110°C und 150°C durchgeführt wird.

7